# EUROPEAN PATENT APPLICATION

(11) **EP 0 890 570 A2**
(43) Date of publication of application: **13.01.1999**
(21) Application number: 98305309.1
(22) Date of filing: 03.07.1998
(51) Int. Cl.: C07C 211/07, C10L 1/22

(54) **Tertiary alkyl primary amines and process for preparing the same**

(30) Priority: 07.07.1997 US 51867
(71) Applicant: ROHM AND HAAS COMPANY, Philadelphia, Pennsylvania 19106-2399 (US)
(72) Inventor: Banavali, Rajiv Manohar, Houston, Texas 77062 (US); Chheda, Bharati Dinkar, Houston, Texas 77068 (US)
(74) Representative: Tanner, James Percival

(57) **Abstract**

A C₈-C₁₀ branched tertiary alkyl primary amine mixture is disclosed which is useful as a multi-functional additive in fuels, lubricants, and dyes. The subject amines are prepared by reacting an acid, a nitrile, a C₈-C₁₀ substrate in the presence of water to form a first intermediate product; further reacting the first intermediate with an acid in the presence of water to form a second intermediate product and neutralizing the second intermediate with a basic compound to form the product.

## Description

The present invention relates to branched tertiary-alkyl ("t-alkyl") primary amines, a process of preparing the same and compositions containing the amines. The amines are useful as multi-functional additives for fuels, lubricants and dyes.

Alkyl amines have found use as fuel oil and lubricant additives. Various, primary, secondary and tertiary amines have been proposed for use in distillate fuel oils as color stabilizers and/or anti-cloggers in concert with other materials. For instance, see U.S. Patent Nos. 2,672,408; 2,684,292; and 2,758,086. Tertiary alkyl primary amines have been found to be particularly useful. Specifically, t-alkyl primary amines, containing carbon chains of C₄, C₇, C₈, mixtures of C₁₂-C₁₅, mixtures of C₁₆-C₁₈ and mixtures of C₁₆-C₂₂ have been proposed and found use as fuel oil stabilizers. For instance, see U. S. Patent No. 2,945,749.

However, noticeably absent from such disclosures are t-alkyl primary amine mixtures in the C₈-C₁₀ range. Aliphatic amines in general, and primary aliphatic amines in particular are widely produced and used, see-*Kirk-Othmer Encyclopedia of Chemical Technology, vol 2, p. 369, 4th edition, 1992, published by John Wiley, NY.* Aliphatic amines are traditionally divided into two classes: (1) lower aliphatic primary amines which typically have a C₁ to C₆ carbon chain length and (2) higher molecular weight amines, typically referred to as fatty amines, typically being in the C₁₂ to C₁₈ range. Lower amines are generally produced from petrochemical feedstocks such as alcohols, e.g., methanol, ethanol, propanol, and butanol. On the other hand, commercially available fatty amines are produced from naturally occurring fatty acids derived from fatty oils, e.g., tallow, coconut, soya, and palm oils. However, partly because of a lack of suitable raw materials, there is a gap in the available amines around the C₉ range.

Moreover, t-alkyl primary amines, which possess special properties over ordinary primary amines also have a gap around the C₉ range. Commercially this gap is important because one can obtain a fine balance of properties including liquidity, hydrophilic-lipophilic balance, and molecular weight optimization.

The current invention fills this gap by providing branched t-alkyl primary amines in the C₈-to C₁₀ range which combine the useful properties derived from molecular weight (C₈-C₁₀) as well as from the branched t-alkyl group. As a combination, the branched C₈-C₁₀ t-alkyl primary amines of the invention are noteworthy in that:
(1) excellent non-polar hydrocarbon miscibility is exhibited (both lower and fatty amines have either poor or complete lack of solubility in non-polar solvents such as mineral oil, kerosene, naphtha, etc);
(2) an ideal balance in oil solubility and water solubility is obtained (fatty amines are somewhat oil soluble, but show little water solubility and lower amines are water soluble, but show little oil solubility);
(3) a wider liquidity range, i.e., shows liquidity even at extreme low temperatures coupled with low volatility at atmospheric temperatures (fatty amines are generally solid at around atmospheric temperatures while lower aliphatic amines tend to be too volatile, thus handling problem exists); and
(4) more amine nitrogen per pound as compared to fatty amines;
(5) higher basicity than either fatty or lower aliphatic primary amines, i.e., pKₐ of 11.5 versus 10.

The branched t-alkyl amine mixtures of the present invention are useful as multi-functional additives in fuels, lubricants and dyes.

In a first aspect of the present invention, there is provided a mixture of branched tertiary alkyl primary amines, comprising at least one of C₈, C₉, and C₁₀ isomers of the formula (1) 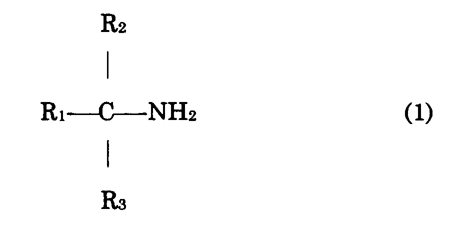 wherein R₁, R₂, and R₃ are each independently, (C₁-C₆) alkyl or substituted alkyl, (C₁-C₆) alkenyl or substituted alkenyl, and wherein in at least 50 % of the isomers at least one of R₁, R₂, and R₃ is a branched carbon chain.

In a second aspect of the present invention, there is provided a process for preparing a mixture of C₈-C₁₀ t-alkyl primary amines, comprising the steps of (A) heating at a temperature in the range from 0°C to 100°C a reaction mixture, comprising an acid, a nitrile, water and a C₈-C₁₀ substrate compound capable of generating a carbonium ion by reaction with the acid, to generate a first reaction intermediate in the reaction mixture; (B) contacting the first reaction intermediate in the reaction mixture with an acid in the presence of water to generate a second reaction intermediate; and (C) neutralizing the second reaction intermediate with a basic compound to form the amine mixture.

As used herein the terminology "(C₁-C₆)" or "(C₁-C₅)" means a straight chain or branched chain alkyl group having from 1 to 6 or 1 to 5 carbon atoms per group. Also, the term "alkene oligomer" means a compound consisting of a linear or cyclic, branched chain of repeating units derived by polymerizing alkene monomers.

Also, the term "major amount" is understood to mean greater than 50 percent by weight and the term "minor amount" is understood to mean less than 50 percent by weight.

As used herein, a carbon at which branching occurs is a secondary (2°) carbon, i.e., a carbon bonded to two other carbon atoms; or a tertiary (3°), i.e., a carbon bonded to three other carbon atoms, *see generally Morrison, R.T. and Boyd, R.N., Organic Chemistry, Prentice Hall, Inc. 6th Ed. 1992, page 92.* Also, the term "primary amine tertiary carbon" refers to a tertiary carbon bonded to an amine nitrogen.

As used herein, the term "strong base" refers to those bases having a pKₐ of greater than or equal to about 8.

Throughout this specification and claims, unless otherwise indicated, references to percentages are by weight, all temperatures by degree centigrade and all pressures are atmospheric.

It is also to be understood that for purposes of this specification and claims that the range and ratio limits, recited herein, are combinable. For example, if ranges of 1-20 and 5-15 are recited for a particular parameter, it is understood that ranges of 1-15 or 5-20 are also contemplated.

The branched t-alkyl primary amines of the present invention are generally characterized as being a complex mixture of at least one of C₈, C₉ and C₁₀ primary amine isomers having a t-alkyl carbon attached to the amine nitrogen and having carbon chains attached to the primary amine tertiary carbon. That is, it is understood that the mixture of branched t-alkyl primary amines may be a mixture of C₈ or C₉ or C₁₀ or C₈ and C₉ or C₉ and C₁₀ or C₈ and C₁₀ as well as C₈, C₉ and C₁₀ isomers. Generally, in some of the isomers, at least one of the carbon chains attached to the primary amine tertiary carbon is a branched carbon chain. That is, the carbon chains attached to the primary amine tertiary carbon may contain secondary and/or tertiary carbons within the chain. This structure is illustrated in Formula 1 above.

In one embodiment, in at least 50 percent, preferably in at least 75 percent, more preferably in at least 90 percent of the isomers at least one of R₁, R₂, and R₃ is a branched carbon chain. In another embodiment, the mixture is at least 50 percent, preferably at least 75 percent, more preferably at least 90 percent C₉ isomers; up to 50 percent, preferably up to 25 percent, more preferably up to 10 percent C₈ isomers; and up to 50 percent, preferably up to 25 percent, more preferably up to 10 percent C₁₀ isomers.

Examples of C₉ isomers which may be present in the amine mixture of the present invention include, but are not limited to, 3,4-dimethyl heptane-4-amine, 3,4-dimethyl heptane-3-amine, 2,3-dimethyl heptane-2-amine, 2,3-dimethyl heptane-3-amine, 2,5-dimethyl heptane-2-amine, 3,6-dimethyl heptane-3-amine, 2,4-dimethylheptane-2-amine, 2,4-dimethyl heptane-4-amine, 3,5-dimethyl heptane-3-amine, 2,6-dimethyl heptane-2-amine, 3-methyl-octane-3-amine, 4-methyl-octane-4-amine, 2-methyl-octane-2-amine, 2,3,5-trimethyl hexane-2-amine, 2,3,5-trimethyl hexane-3-amine, 2,4,5-trimethyl hexane-3-amine, 2,3,4-trimethyl hexane-2-amine, 2,3,4-trimethyl hexane-3-amine, 3,4,5-trimethyl hexane-3-amine, 2,5,5-trimethyl hexane-2-amine, 3,4,4-trimethyl hexane-3-amine, and 3,5,5-trimethyl-3-hexane-3-amine. C₉ isomers other than those of Formula 1 may also be present. Examples include, but are not limited to, n-nonylamine, and iso-nonyl amine.

Examples of C₈ isomers which may be present in the amine mixture of the present invention include, but are not limited to, 2,3-dimethyl hexane-3-amine, 2,3-dimethyl hexane-2-amine, 2,4-dimethyl hexane-2-amine, 2,4-dimethyl hexane-4-amine, 2,5-dimethyl hexane-2-amine, 2-methylheptane-2-amine, 3-methylheptane-3-amine, 4-methyl heptane-4-amine. C₈ isomers other than those of Formula 1 may also be present. Examples include, but are not limited to, n-octylamine, and t-octyl amine.

Examples of C₁₀ isomers which may be present in the amine mixture of the present invention include, but are not limited to, 2,3-dimethyl octane-2-amine, 2,3-dimethyl octane-3-amine, 2,4-dimethyl octane-2-amine, 2,4-dimethyl octane-4-amine, 2,5-dimethyl octane-2-amine, 2,5-dimethyl octane-5-amine, 2,6-dimethyl octane-2-amine, 2,6-dimethyl octane-6-amine, 2,7-dimethyl octane-2-amine, 3,4-dimethyl octane-3-amine, 3,4-dimethyl-octane-4-amine, 3,5-dimethyl-octane-3-amine, 3,5-dimethyl-octane-5-amine, 3,6-dimethyl octane-3-amine, 3,7-dimethyl octane-7-amine, 4,5-dimethyl octane-4-amine, 4,6-dimethyl octane-4-amine, 4,6-dimethyl octane-6-amine, 4,7-dimethyl octane-4-amine, 4,7-dimethyl octane-7-amine, 2-methyl nonane-2-amine, 3-methyl nonane-3-amine, 4-methyl nonane-4-amine, and 5-methyl nonane-5-amine. C₁₀ isomers other than those of Formula 1 may also be present. Examples include, but are not limited to, n-decylamine, and iso-decyl amine.

The amine mixtures of the present invention are strong bases, especially in non-polar organic media. The amine mixtures have a pKₐ which is generally greater than the pKₐ of other types of C₈-C₁₀ amines. Typically, conventional primary amines in this range have a pKₐ from 9-11. For instance, iso-nonyl amine and n-nonyl amine both have a pKₐ of 10. The pKₐ of the amine mixtures of the present invention are generally from 10.5 to 12, preferably 10.8 to 12.0, more preferably from 11.0 to 12.0.

The preferred amine mixture is one prepared from propylene trimer.

As recited above, a process for making a mixture of C₈-C₁₀ t-alkyl primary amines is also disclosed. Substrate compounds useful in the method of the present invention are those known as substrates for the Ritter reaction and include, for example, alcohols, alkenes, aldehydes, ketones, ethers, *see, generally, L. I. Krimen and D. J. Cota, "The Ritter Reaction", Organic Reactions, Vol. 17, 1969, pp. 213-325.*

In one embodiment, the substrate is an alkene, preferably, a C₈-C₁₀ alkene or mixture of C₈-C₁₀ alkenes according to the structural formula (2): 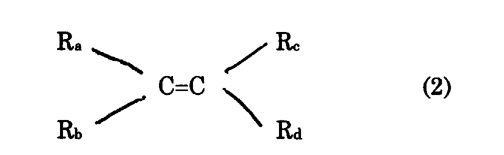 wherein, Rₐ and R_{b} are each independently (C₁-C₆) alkyl, substituted (C₁-C₆) alkenyl; and R_{c} and R_{d} are each independently hydrogen or (C₁-C₆) alkyl, substituted (C₁-C₆) alkyl, (C₁-C₆) alkenyl or substituted (C₁-C₆) alkenyl.

Suitable examples of (C₁-C₆) alkyl include, but are not limited to, methyl, ethyl, n-propyl, isopropyl, n-butyl, isobutyl, tert-butyl, n-pentyl, isopentyl, neopentyl, n-hexyl, etc. Suitable examples of (C₁-C₆) substituted alkyl include, but are not limited to, methyl, ethyl, n-propyl, isopropyl, n-butyl, isobutyl, tert-butyl, n-pentyl, isopentyl, neopentyl, n-hexyl, etc., substituted with hydroxy, halide, and nitro.

Suitable examples of (C₁-C₆) alkenyl include, but are not limited to, ethenyl, n-propenyl, isopropenyl, n-butenyl, isobutenyl, tert-butenyl, n-pentenyl, isopentenyl, neopentenyl, n-hexenyl, etc. Suitable examples of (C₁-C₆) substituted alkyl include, but are not limited to, ethenyl, n-propenyl, isopropenyl, n-butenyl, isobutenyl, tert-butenyl, n-pentenyl, isopentenyl, neopentenyl, n-hexenyl, etc., substituted with hydroxy, halide, or nitro groups.

Examples of useful alkenes according to the present invention include, but are not limited to, 3,4-dimethyl heptene, 2,3-dimethyl heptene, 2,5-dimethyl heptene, 2,4-dimethyl heptene, 3,5-dimethyl heptene, 2,6-dimethyl heptene, 2-methyl octene, 3-methyl octene, 4-methyl octene, 2,3,5 trimethyl hexene, 2,3,4 trimethyl hexene, 2,5,5 trimethyl hexene, 3,5,5 trimethyl hexene, 2,3-dimethyl hexene, 2,4-dimethyl hexene, 2,5-dimethyl hexene, 2-methyl heptene, 3-methyl heptene, 4-methyl heptene, 2,3-dimethyl octene, 2,4-dimethyl octene, 2,5-dimethyl octene, 2,6 dimethyl octene, 2,7-dimethyl octene, 3,4-dimethyl octene, 3,5 dimethyl octene, 3,7-dimethyl octene, 4,5-dimethyl octene, 4,6 dimethyl octene, 4,7-dimethyl octene, 2-methylnonene, 3-methylnonene, 4-methylnonene, 5-methylnonene, alkene oligomers, and mixtures thereof. Suitable alkene oligomers include, but are not limited to, propylene trimer, butylene dimer, and isobutylene dimer.

In a preferred embodiment, the substrate compound is an alkylene oligomer, more preferred propylene trimer.

In one embodiment, the acid used is a strong acid (HA) having a pKₐ of less than 5, preferably less than 3, more preferably less than 2. Examples of strong acids useful in the process of the present invention, include, but are not limited to, inorganic acids such as, hydrochloric acid, phosphoric acid, perchloric acid, sulfuric acids; organic acids such as, for example formic acid, methane sulfonic acid, p-toluene sulfonic acid; and strongly acidic ion exchange resins such as, for example, Amberlyst® 15 resin (Rohm and Haas Company, Philadelphia, Pa.).

In a more preferred embodiment, the acid is a concentrated aqueous solution of sulfuric acid that includes from 60 percent to 100 percent by weight sulfuric acid.

In one embodiment, the reaction mixture includes from 0.2 moles to 4 moles, preferably from 0.5 moles to 3 moles, more preferably 1 mole to 2 moles of the acid per mole of reactive sites of the substrate compound, wherein the number of moles of reactive sites of the substrate compound equals the product of the number of moles of substrate compound multiplied by the number of reactive sites per molecule of the substrate compound.

The nitrile may be any nitrile which will react with the acid and substrate in the presence of water to form the first reaction intermediate. In one embodiment the nitrile is a nitrile of the formula (3)

R-C ≡ N (3)

wherein R is H, (C₁-C₅) alkyl or substituted alkyl, vinyl, phenyl or substituted phenyl. Examples of suitable alkyl groups, include but are not limited to, methyl, ethyl, n-propyl, isopropyl, n-butyl, isobutyl, t-butyl, n-pentyl, isopentyl, and t-pentyl.

Examples of nitriles which may be used in the process of the present invention include, but are not limited to, hydrogen cyanide, acetonitrile, acrylonitrile, butylnitrile, benzonitrile, and p-tolunitrile. Preferably, the nitrile is hydrogen cyanide. The nitrile may be added directly to the reaction mixture. Alternatively, the nitrile may be generated in situ by adding a compound which forms a nitrile under the reaction conditions of the present invention for the first reaction mixture.

In a preferred embodiment, hydrogen cyanide is added directly to the reaction mixture as the nitrile. In an alternative embodiment, a compound which generates hydrogen cyanide under the reaction conditions of the present process is added to the reaction mixture and hydrogen cyanide is generated in situ. Examples of hydrogen cyanide-generating compounds include, but are not limited to, cyanide salts such as, for example, sodium cyanide, and potassium cyanide.

In one embodiment, the reaction mixture includes from 1 mole to 10 moles, preferably from 1 mole to 5 moles, more preferably from 1 mole to 1.5 moles, nitrile or an equivalent amount of nitrile-generating compound per mole of reactive sites of the substrate.

The reaction mixture of the first step of the process includes from 0.8 mole to 10 moles, preferably from 1.0 mole to 5 moles, more preferably from about 1 mole to about 3 moles water per mole of reactive sites of the substrate.

The reaction mixture is heated at a temperature from 0°C to 120°C, preferably from 25°C to 90°C, more preferably, from 30°C to about 60°C during the first process step. In one embodiment, the substrate compound is charged to the reaction vessel and the acid and nitrile are fed into the reaction vessel according to feed rate profiles that allow heating of the reaction mixture by reaction exotherm to a selected temperature. That is, by controlling the rate of addition of acid and nitrile the reaction temperature is maintained within the desired temperature range.

The reaction mixture is generally maintained at a pressure from 1 atmosphere to 10 atmospheres, preferably from 1 atmosphere to 5 atmospheres, more preferably at 1 atmosphere, during the first process step.

In the second process step, the first reaction intermediate is treated with an acid in the presence of water. The reaction mixture of the second step of the process includes from 0.5 mole to 20 moles, preferably from 1 mole to 15 moles, more preferably from 1 mole to 10 moles water per mole of reactive sites of the substrate compound.

The second reaction intermediate is not isolated from the reaction mixture. While not wishing to be bound by theory, it is believed that in the preferred embodiment wherein the substrate is an alkene according to the structural formula (1), the second reaction intermediate is an ammonium salt having the structural formula (4): 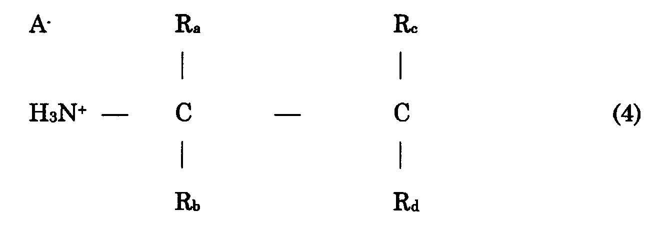 wherein, A- is the residue of the acid (HA) used in the first process step, and Rₐ, R_{b}, R_{c}, and R_{d} are each as defined above.

Acids used in the second process step are those disclosed above as being suitable for use as the acid utilized in the first process step, with concentrated sulfuric acid being preferred as (HA).

The reaction mixture is heated to a temperature of from 40°C to 150°C., preferably from 50°C to 130°C., more preferably 60°C to 110°C, during the second process step. The reaction mixture is maintained at a pressure from 0.1 to 2 atmospheres, preferably 0.5 to 1.5 atmospheres, more preferably at 1 atmosphere, during the second process step.

In a third process step, the second reaction intermediate is neutralized generally to pH 9 or higher, with a basic compound to form the amine mixture of formula (1). In one embodiment, the basic compound is a strong base. Such bases include, but are not limited to, ammonium hydroxide, sodium hydroxide, potassium hydroxide, and aqueous ammonia, In a preferred embodiment, the strong base is sodium hydroxide or aqueous ammonia, more preferably an aqueous ammonia solution having a pKₐ of at least 8.0.

In a preferred embodiment, the amine product is recovered and purified from the reaction mixture by removing and distilling the organic layer to provide the amine product. In another embodiment, the separation of the organic layer from the aqueous layer may, optionally, take place concurrently with the second process step.

In an optional step one or more of C₈, C₉, or C₁₀ isomers may be removed by, for instance, distillation or other means known in the art so that the resultant amine mixture may be a mixture of C₈ or C₉ or C₁₀ or C₈ and C₉ or C₉ and C₁₀ or C₈ and C₁₀ as well as C₈, C₉ and C₁₀ isomers.

The amine mixtures of the present invention are useful as multi-functional additives in fuel oils, lubricants and dyes. The multi-functional properties make them especially suitable as stabilizers for fuels. Accordingly, the amine mixtures of the present invention may be added to fuels to provide stabilization. While not wishing to be bound by theory, it is believed stabilization occurs because of the multi-functional properties imparted by the branched t-alkyl amines. Oxidation and corrosion inhibition, as well as dispersancy and improved demulsibility properties which are imparted by the amine mixtures of the present invention combine to stabilize the fuel. The present amine mixture serves not only to mitigate the oxidative processes, e.g., by peroxide decomposition, ongoing in the fuel, but also by forming fuel soluble salts with acidic oxidation by-products and/or complexing with metals and other species promotes suspension of such by-products. Thus, color and sludge formation are minimized. Accordingly, the branched t-alkyl primary amine mixtures of the present invention act to prevent deterioration of the fuel to which it is added as well as act to resolve problems caused by fuel deterioration which has occurred.

In one embodiment, a fuel composition is disclosed including (A) a major amount of fuel; and (B) a minor amount of an amine mixture according to formula 1 effective to impart multi-functional properties to the fuel oil.

Generally, the fuel is present in a major amount in the fuel composition. In a preferred embodiment, the fuel oil is present in an amount of at least 75% by weight, more preferably 90% by weight. The amine mixture according to formula 1 is generally present in a minor amount which is effective to impart multi-functional properties to the fuel. In one embodiment, the amine mixture is present at a concentration from 1 to 300, preferably from 5 to 100, more preferably from 10-50 pounds per 1000 barrels of fuel.

The fuels useful in the present invention are generally any fuel which may suffer deterioration during storage. In a preferred embodiment, the fuels are hydrocarbon fractions having an initial boiling point of at least 100°F and an end point not higher than 750°F, which boil substantially continuously throughout their distillation range. Such fuels are generally known as middle distillate fuels. Examples of middle distillate fuels which may be used in the fuel compositions of the present invention include, but are not limited to, distilled oils, furnace oils, diesel oils, jet fuels, and residual fuels such as bunker fuels, marine diesel fuels, railroad diesel fuels, etc.

The amine mixture according to formula (1) is as described above with the amine mixture prepared from propylene trimer being preferred.

Also contemplated is a method of stabilizing fuels, comprising introducing into said fuel an amine mixture according to figure 1 in an amount effective to stabilize the fuel. The fuels and amine mixture according to formula (1) are as described above, with middle distillate fuels and the amine mixture prepared from propylene trimer being preferred. In one embodiment, the amine mixture according to formula 1 is introduced at a concentration from 1 to 300, preferably from 5 to 100, more preferably from 10-50 pounds per 1000 barrels of fuel oil.

The following Examples are provided as an illustration of the present invention.

### EXAMPLE 1

### Preparation of C₈-C₁₀ branched tertiary alkyl primary amine mixture

A 1 liter 4-necked reaction vessel was equipped with heating mantle, a thermometer, a mechanical stirrer, a reflux condenser, a hydrogen cyanide feed burette and an acid feed burette. The vessel was charged with 126 g of propylene trimer, 122.5 g of sulfuric acid, 22.5 g water, and 33.2 g of hydrogen cyanide. The reaction mixture was stirred while maintaining the temperature between 25-50°C for a total of 2 hours.

After the 2 hour period, the burettes were removed from the reaction vessel and the reaction vessel was equipped with a Dean-Stark trap fitted with a reflux condenser. Water, 183 g., was added to the reaction vessel and the mixture was heated and refluxed with agitation for 3.5 hr. After 3.5 hr. the reaction mixture was cooled to room temperature. The Dean-Stark trap and condenser assembly was then replaced by an aqueous ammonia addition funnel. The reaction mixture was neutralized by slowly adding aqueous ammonia to the reaction mixture while cooling. Stirring was discontinued and the reaction mixture was allowed to separate into an organic liquid layer and an aqueous liquid layer. The layers were then separated to provide an organic layer of 122.9 g. The organic layer was distilled and the product cut was collected at 160-174°C to provide 118.2 g of the amine product (81.4% of theoretical yield). The amine product had a pKₐ of 11.5, a boiling point of 170°C and a freezing point of-103°C.

The composition of this material was determined by gas chromatography using a Hewlett-Packard Co. HP5990 Series II GC with flame ionization detector and an RTX-5 capillary column 30M length x .53 mm diameter x 1µ film thickness available from Restek Company. The chromatographic analysis revealed a complex amine mixture containing, 96.01 percent C₉ amine isomers, 0.02 percent C₈ amine isomers and 3.97 percent C₁₀ amine isomers.

### EXAMPLES 2-10

### Preparation of other branched tertiary alkyl primary amine mixtures

Additional mixtures of branched tertiary alkyl primary amines are prepared according to the procedure of Example 1, except that the substrate is an alkene as defined in Formula (2) and Table 1.

**Table 1**

| **Example** | **R**_{**a**} | **R**_{**b**} | **R**_{**c**} | **R**_{**d**} |
|---|---|---|---|---|
| 2 | methyl | isobutyl | H | n-propyl |
| 3 | H | isobutyl | methyl | ethyl |
| 4 | ethyl | methyl | H | n-pentyl |
| 5 | methyl | methyl | H | isopentyl |
| 6 | methyl | n-propyl | H | isobutyl |
| 7 | methyl | methyl | H | 2-hexyl |
| 8 | ethyl | methyl | H | n-butyl |
| 9 | n-propyl | methyl | H | n-pentyl |
| 10 | H | 3,3-dimethyl butyl | methyl | methyl |

### EXAMPLES 11-14

### Fuel storage test

Fuel composition samples of catalytically cracked furnace oil (No. 2 fuel) containing various concentrations of additives as indicated in Table I were prepared. Fuel storage tests were performed as follows. The prepared fuel compositions were stored in 4-ounce fuel sample bottles in a large oven at 43°C. At intervals, optical density measurements were made, using a Lambda 2 UV/VIS Spectrometer from Perkin Elmer Corp. on samples before and after filtering a small portion of a vigorously shaken sample through a Corning 30F fritted glass crucible. The unused portion was then returned to the oven for further aging. By graphical or other interpolation, the failure time was determined as the number of days to a stated level of optical density. The two levels quoted here are: 1) a difference [ΔOD] of 0.12 between unfiltered and filtered portions and 2) an OD value of 1.00 [TOD] for the unfiltered sample. The failure time ratio is the ratio of failure time of the blend to that of the untreated base stock run as control. The results are shown in Table II.

**Table II**

| **Example** | **Additive** | **Conc. lbs/1000 bbl** | **Days to failure (1.0 mg insolubles in 100 mL**) |
|---|---|---|---|
| 11 | none | -- | 29 |
| 12 | Primene® 81-R¹ | 15 | 70 |
| 13 | Primene® TOA² | 15 | 70 |
| 14 | Amine mixture of Example 1 | 15 | 90 |

| | | | |
|---|---|---|---|
| ¹ A C₁₂-C₁₄ mixture of tertiary primary amines available from Rohm and Haas Company of Philadelphia, Pa. | | | |
| ².t-octyl primary amine available from Rohm and Haas Company of 10 Philadelphia, Pa. | | | |

Table II indicates that the branched t-alkyl primary amines of the present invention impart stability to fuels which is superior to conventional amines known in the art.

### EXAMPLES 15-23

### Diesel fuel oxidative stability test

Fuel composition samples of diesel oil (Gulf Coast - 0.4% Sulfur, Howell) containing various concentrations of additives as indicated in Table m were prepared. Diesel Fuel oxidative stability tests were performed using a Modified ASTM 2274 Test Method. The ASTM method was modified by changing the test duration from 16 hours to 40 hours. The oxidation stability of the fuel compositions is shown in Table III as the amount of insolubles present.

**Table III**

| **Example** | **Additive** | **Conc.(ppm)** | **Insolubles mg/100 mL** |
|---|---|---|---|
| 15 | none | -- | 2.5 |
| 16 | Primene® 81-R¹ + Acryloid 917T(70/30)² | 20 | 1.3 |
| 17 | Amine mixture of Example 1 | 15 | <0.1 |
| 18 | Primene® 81-R¹ ² | 15 | 1.2 |
| 19 | Primene® JM-T³ | 15 | 1.4 |
| 20 | Primene® TOA⁴ | 15 | 0.4 |
| 21 | 2-ethylhexylamine | 15 | 2.2 |
| 22 | n-nonylamine | 15 | 1.9 |
| 23 | iso-nonylamine | 15 | 2.1 |

| | | | |
|---|---|---|---|
| ¹ A C₁₂-C₁₄ mixture of tertiary primary amines available from Rohm and Haas Company of Philadelphia, Pa. | | | |
| ² A alkyl methacrylate copolymer in solvent refined neutral oil available from 5 Rohm and Haas Company of Philadelphia, Pa. | | | |
| ³.A C₁₆-C₂₂ mixture of tertiary primary amines available from Rohm and Haas Company of Philadelphia, Pa. | | | |
| ^{4.}t-octyl primary amine available from Rohm and Haas Company of Philadelphia, Pa. | | | |

Table III indicates that the branched t-alkyl primary amines of the present invention impart oxidative stability to fuels which is superior to known conventional amines and amine mixtures.

## Claims

1. A mixture of branched tertiary-alkyl primary amines, comprising: at least one of C₈, C₉, and C₁₀ isomers of the formula: 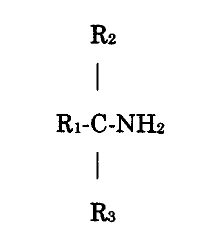 wherein R₁, R₂, and R₃ are each independently, (C₁-C₆) alkyl or substituted alkyl, (C₁-C₆) alkenyl or substituted alkenyl, and wherein in at least 50 % of the isomers at least one of R₁, R₂, and R₃ is a branched carbon chain.

2. The mixture of claim 1, having at least 50 percent C₉ isomers, up to 50 percent C₈ isomers and up to 50 percent C₁₀ isomers.

3. The mixture of claim 1, having a pKₐ of from 10.5 to 12.0.

4. A process for preparing a mixture of C₈-C₁₀ branched tertiary-alkyl primary amines, comprising the steps of:
(A) heating a reaction mixture at a temperature in the range of 0°C to 120°C, comprising an acid, a nitrile, water and a C₈-C₁₀ substrate compound capable of generating a carbonium ion by reaction with the acid, to generate a first reaction intermediate in the reaction mixture;
(B) contacting the first reaction intermediate in the reaction mixture with an acid in the presence of water to generate a second reaction intermediate; and
(C) neutralizing the second reaction intermediate with a basic compound to form the amine mixture.

5. The process according to claim 4, wherein the substrate is a compound of the formula 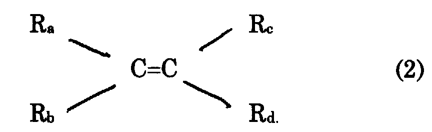 wherein, Rₐ and R_{b} are each independently (C₁-C₆) alkyl, substituted (C₁-C₆) alkenyl; and R_{c} and R_{d} are each independently hydrogen or (C₁-C₆) alkyl, substituted (C₁-C₆) alkyl, (C₁-C₆) alkenyl or substituted (C₁-C₆) alkenyl.

6. The process according to claim 4, wherein the substrate is propylene trimer, the nitrile is hydrogen cyanide, the acid is a concentrated aqueous solution of sulfuric acid that includes from 60 percent to 100 percent by weight sulfuric acid and the basic compound is an aqueous ammonia solution having a pKₐ of at least 8.0.

7. A fuel composition, comprising:
(A) a major amount of fuel; and
(B) an amine mixture according to claim 1 present in a minor amount amount effective to impart multi-functional properties to the fuel.

8. The fuel composition according to claim 7, wherein the amine mixture is present at a concentration from 1 to 300 pounds per 1000 barrels of fuel.

9. A method of stabilizing fuels, comprising: introducing into the fuel an amine mixture according to claim 1 in an amount effective to stabilize the fuel.

10. The method of claim 9, wherein the amine mixture is introduced in an amount of from 1 to 300 pounds per 1000 barrels of fuel.
